# FASCICULE DE BREVET EUROPEEN

(11) **EP 0 773 746 B1**
(45) Date de publication et mention de la délivrance du brevet: **08.09.1999**
(21) Numéro de dépôt: 94900202.6
(22) Date de dépôt: 17.11.1993
(51) Int. Cl.: A61B 17/70

(54) **DISPOSITIF DE FIXATION SUR UNE TIGE POUR ORTHOPEDIE RACHIDIENNE**
STABBEFESTIGUNGSVORRICHTUNG FÜR SPINALE ORTHOPÄDISCHE ZWECKE
ROD ATTACHMENT DEVICE FOR SPINAL ORTHOPAEDICS

(30) Priorité: 18.11.1992 FR 9213868
(43) Date de publication de la demande: 21.05.1997
(73) Titulaire: EUROSURGICAL, F-62750 Loos-en-Gohelle (FR)
(72) Inventeur: ROKEGEM, Pascal, F-62223 Saint-Laurent-Blangy (FR); STEIB, Jean-Paul, Frédéric, F-67100 Strasbourg (FR)
(74) Mandataire: Schmitt, John
(86) Numéro de dépôt international: FR9301128
(87) Numéro de publication internationale: WO9410929

(56) Documents cités:
- EP-A- 0 392 927
- EP-A- 0 443 894
- DE-A- 3 924 050
- FR-A- 2 638 632

## Description

La présente invention se rapporte à un dispositif de fixation sur une tige, d'un organe tel que vis, crochet ou analogue, notamment pour une instrumentation d'orthopédie rachidienne, du type décrit dans le préambule de la revendication 1.

On connaît déjà diverses instrumentations ou matériel posés orthopédiques, tels que ceux connus sous l'appellation HARRINGTON, et qui permettent de redresser et d'étayer le rachis ou colonne vertébrale d'un patient, par exemple atteint d'une scoliose.

Un dispositif de fixation du type décrit dans le préambule de la revendication 1 est connu par le document FR-A-2 638 632. Dans ce dispositif connu, la douille intermédiaire a une forme générale cylindrique et présente une section droite au plus égale à un demi-cercle dont le diamètre intérieur est de l'ordre de grandeur du diamètre de la tige. L'organe d'instrumentation est fixé sur la tige à l'aide d'une vis de serrage qui agit directement sur la tige en repoussant celle-ci dans le fond du corps en forme de U solidaire dudit organe, la douille intermédiaire étant retenue par ce corps en forme de U.

On a également proposé dans l'art antérieur des dispositifs de fixation d'un organe sur une tige comportant des aspérités de surface, telles que moletage ou guillochage. Dans ces dispositifs, les moyens de serrage sont constitués par une vis de pression montée sur la douille et apte à exercer une force radiale sur la tige, afin de rendre celle-ci solidaire de la douille en écrasant les aspérités, pour augmenter l'adhérence entre celle-ci et la tige.

Toutefois, avec les dispositifs connus, le montage des douilles sur la tige correspondante constitue une manoeuvre compliquée avant la mise en place du matériel, ce qui rend fastidieuse et augmente indésirablement la durée de l'opération chirurgicale nécessaire à l'implantation de ce matériel.

De plus, si la tige comporte des usinages tels que filetages ou encoches, comme c'est le cas par exemple dans les instrumentations HARRINGTON, les efforts appliqués sur les organes génèrent des concentrations de contraintes au niveau des usinages de la tige, qui peuvent provoquer sa rupture. Par ailleurs, si la tige comporte des aspérités de surface, celle-ci a tendance à se mater au niveau de ses zones de contact avec la douille ou la vis, de sorte que le serrage et donc l'immobilisation de l'organe sur la tige s'altèrent fréquemment après l'implantation du matériel. Dans un cas comme dans l'autre, ces défauts peuvent être préjudiciables pour la tenue des organes d'ancrage et être à l'origine d'une modification de la géométrie de l'instrumentation. Cette modification de géométrie provoque une perte de correction ou de réduction et une mobilisation des organes d'ancrage sous l'effet des contraintes imposées par le patient.

L'effet secondaire de cette mobilisation peut être à l'origine d'une corrosion par frottement dite "fretting corrosion".

La présente invention a pour but de proposer un dispositif de fixation qui ne présente pas les inconvénients de l'état de la technique, qui viennent d'être énoncés, et assure une excellente tenue de la tige dans sa position d'immobilisation sous l'effet de la vis de serrage.

Pour atteindre ce but, le dispositif de fixation selon l'invention comporte les caractéristiques qui sont énoncées dans la partie caractérisante de la revendication 1.

D'autres caractéristiques avantageuses sont énoncées dans les revendications dépendantes 2 à 13.

L'invention sera mieux comprise et d'autres buts, caractéristiques, détails et avantages de celle-ci apparaîtront plus clairement au cours de la description explicative qui suivra faite en référence aux dessins annexés donnés uniquement à titre d'exemple et illustrant plusieurs modes de réalisation de l'invention et dans lesquels :
La figure 1 est une vue en perspective de la face postérieure d'un tronçon de rachis sur lequel une instrumentation orthopédique d'étayement équipée de dispositifs conformes à l'invention est implantée.
La figure 2 est une vue en perspective et éclatée d'une premier mode de réalisation d'un dispositif de fixation selon l'invention.
La figure 3 représente une variante de réalisation d'un organe d'ancrage selon l'invention.
Les figures 4 et 5 représentent un organe équipé d'un dispositif suivant une autre variante de réalisation d'un corps d'ancrage selon l'invention.
La figure 6 est une vue en perspective et éclatée d'un deuxième mode de réalisation d'un dispositif de fixation selon l'invention.
La figure 7 est une vue en perspective et éclatée d'un troisième mode de réalisation d'un dispositif de fixation selon l'invention.
Les figures 8 et 9 représentent deux étapes successives de la fixation sur une tige d'un organe d'ancrage du mode de réalisation selon la figure 7.
Les figures 10a,10b et 10c montrent trois phases successives de l'assemblage du mode de réalisation selon la figure 7.

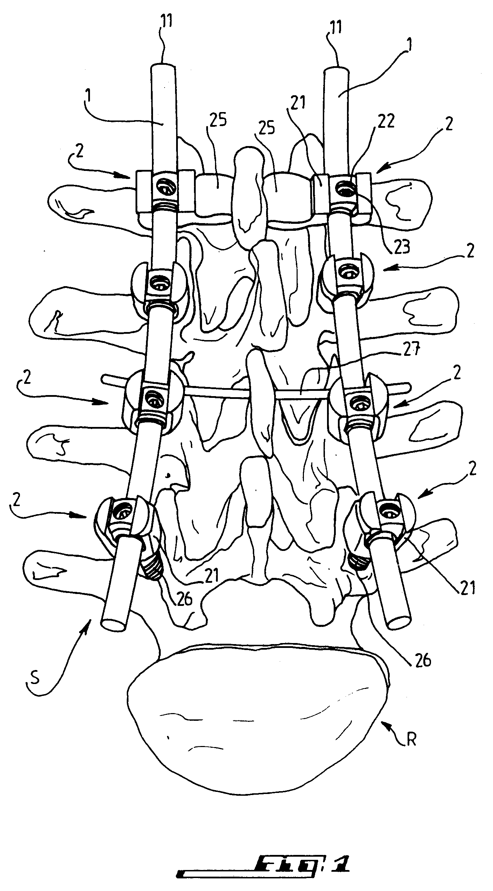

Sur la figure 1, on voit un tronçon de rachis ou colonne vertébrale R sur lequel est implanté un matériel ou instrumentation orthopédique S. Comme expliqué précédemment, le matériel S comprend deux tiges d'étayement 1, dont les axes longitudinaux s'étendent le long de la face postérieure du rachis R, et de part et d'autre de celui-ci. Les tiges 1 sont de préférence en acier chirurgical doux et sont cintrées afin de présenter une courbure choisie en fonction du rachis à étayer R.

Divers organes sont fixés sur chaque tige 1 d'une part, et d'autre part rendus solidaires d'une vertèbre du rachis R, ou d'un dispositif fixé sur l'autre tige 1 de l'instrumentation S.

Dans le premier cas, les organes seront des vis, crochets ou analogues permettant l'ancrage de l'instrumentation S sur le rachis R, tandis que dans le second, les organes agiront comme des entretoises reliant les deux tiges 1.

Chacun de ces organes est immobilisé sur la tige correspondante 1 à l'aide d'un dispositif de fixation propre. Sur les figures, chaque ensemble constitué par un organe d'ancrage ou formant entretoise et par son dispositif de fixation est désigné de manière générale par la référence numérique 2.

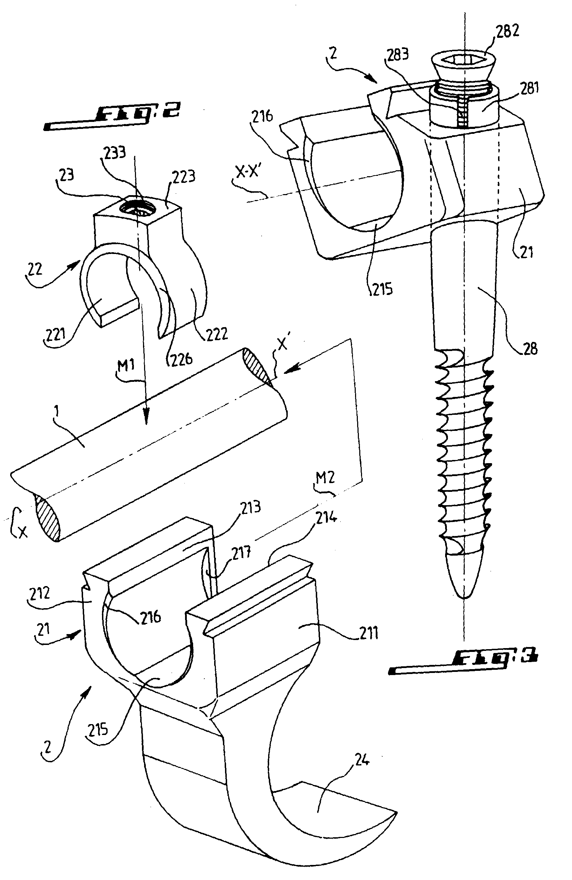

En se reportant maintenant à la figure 2, on voit que le dispositif de fixation de chaque ensemble 2 sur la tige 1 comprend un corps 21 en forme de "U", dont est solidaire ledit organe, et entre les branches duquel la tige 1 correspondante peut être montée "radialement", c'est-à-dire suivant la direction de l'un de ses rayons et sans devoir y être enfilée. On précisera également ici que c'est la section transversale du corps 21, perpendiculaire à l'axe longitudinal X-X' de la tige 1 correspondante, qui présente sensiblement la forme d'un "U".

De plus, une douille intermédiaire 22 peut être montée sur la tige 1 et venir se loger par coulissement entre les branches du "U" du corps 21. Des moyens de serrage 23 sont prévus sur chaque douille 22, et solidaires de celle-ci de façon à pouvoir appliquer sur la tige 1 une pression radiale de serrage.

Conformément à l'invention, les tiges 1 utilisées ont une section transversale circulaire et une surface externe lisse, contrairement aux tiges employées dans l'art antérieur qui étaient soit usinées, soit pourvues d'aspérités de surface.

D'autre part, chacune des douilles 22 conformes à l'invention présente, au lieu d'être en forme de chemise cylindrique comme c'était le cas dans les dispositifs connus, sensiblement la forme d'une bague fendue ou cavalier. On voit bien sur la figure 2 que la bague 22 comporte deux ailes latérales arquées 221 et 222, ainsi qu'une protubérance prismatique 223, en saillie vers l'extérieur de la bague et traversée radialement par un perçage débouchant et taraudé 233. C'est dans ce perçage 233 qu'est montée une vis sans tête 23 constituant les moyens de serrage expliqués plus haut.

A l'opposé du perçage 233, une fente axiale ou orientée suivant X-X' est formée dans la bague 22, de part en part de celle-ci. Autrement dit, le perçage 233 et la fente axiale de la bague 22 sont disposés en regard l'un de l'autre suivant un diamètre de la tige 1 sur laquelle cette bague doit être montée. La distance entre les rebords axiaux en vis-à-vis des ailes latérales 221 et 222 qui définissent la fente axiale de la bague 22, c'est-à-dire la dimension perpendiculairement à X-X' de cette fente, est plus petite que le diamètre de la tige 1 correspondante Toutefois, les ailes sont élastiquement déformables, jusqu'à ce que la fente puisse être traversée par la tige 1.

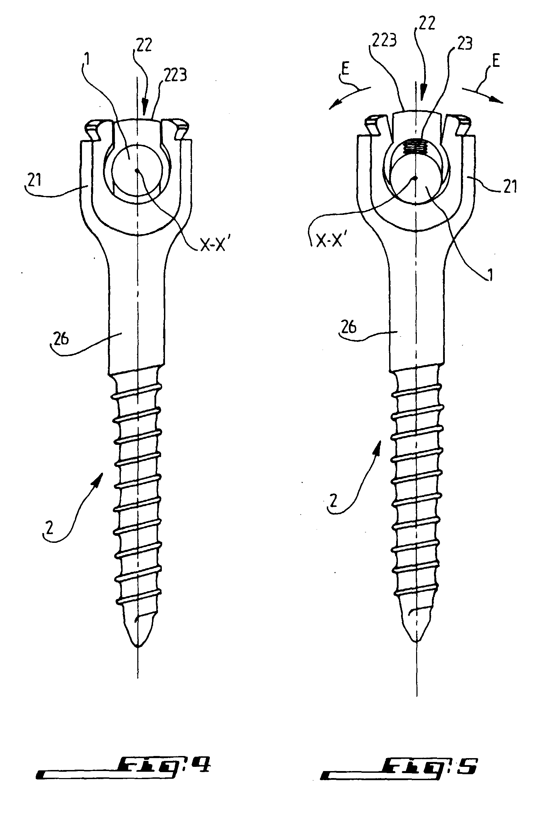

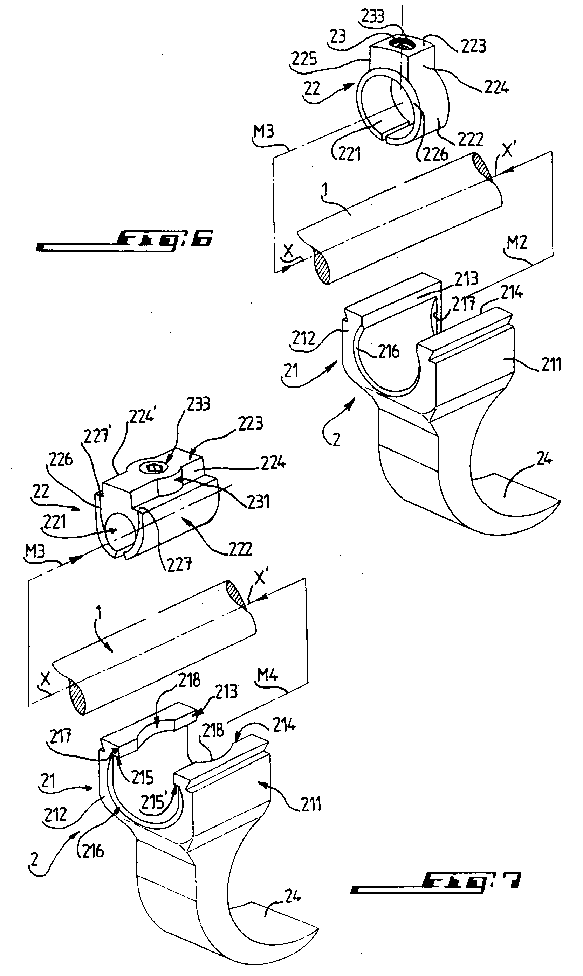

Conjointement, les ailes latérales de la bague 22 définissent une surface interne sensiblement cylindrique et dont la section correspond à peu près à celle de la tige 1. Aussi, puisque les ailes latérales sont élastiquement déformables, on comprend que la bague 22 peut être montée sur la tige 1 radialement à travers ladite fente axiale, comme indiqué par la flèche M1 sur la figure 2. En fait, les ailes 221 et 222 peuvent s'écarter latéralement et glisser sur la périphérie de la tige 1, jusqu'à ce que la bague 22 vienne dans la position visible sur la figure 4. Dans cette position, les ailes latérales 221 et 222 enserrent élastiquement ou immobilisent par "clippage" la tige 1 à l'intérieur de la bague fendue 22, tout en permettant leur coulissement axial relatif.

Sur les figures 2 à 5, on voit que les branches latérales du "U" du corps 21, qui sont respectivement désignées en 211 et 212, définissent ensemble un logement dont la forme correspond sensiblement à celle de la bague ou cavalier 22. Plus spécialement, la surface interne de la branche 211 a une forme généralement cylindrique qui correspond à peu près au profil extérieur de l'aile latérale 221 du cavalier 22. Similairement, l'aile et la branche latérales 222 et 212 présentent des profils respectivement interne et externe correspondants. Ainsi, quand la bague 22 est "clippée" ou engagée élastiquement sur la tige 1, celle-ci peut être guidée à coulissement suivant X-X', à l'intérieur du logement défini par le "U" du corps 21.

En outre, les extrémités en vis-à-vis des branches latérales 211 et 212 définissent respectivement deux surfaces planes 213 et 214, parallèles l'une à l'autre ainsi qu'à l'axe X-X'. Ces surfaces planes 213 et 214 du corps 21 peuvent collaborer avec des surfaces correspondantes 224 et 224' de la protubérance prismatique 223 de la bague 22, afin de guider cette dernière suivant X-X' et d'empêcher toute rotation de la bague à l'intérieur du corps 21.

Sur les figures 2 et 3, on remarque que le fond du logement défini par le "U" du corps 21 comporte un évidement 215, orienté axialement, à savoir suivant X-X'. Cet évidement 215 est diamétralement opposé à l'espace s'étendant entre les surfaces 213 et 214 du corps, et présente en section transversale une forme concave en arc de cercle, à la façon d'une gouttière. Le diamètre du cercle auquel correspond l'arc de la gouttière 215 est sensiblement égal au diamètre de la tige 1.

Bien que ceci ne soit pas illustré, la surface concave de la gouttière 215 est de préférence usinée de manière à présenter des stries transversales (c'est-à-dire perpendiculaires à X-X') et/ou à posséder un état de surface rugueux. Ceci permet d'accroître l'adhérence par frottement entre le corps 21 et la tige 1, et donc d'améliorer l'immobilisation de cette dernière, notamment suivant sa direction longitudinale X-X'.

On notera également ici que l'une au moins des extrémités opposées suivant X-X' de la bague fendue 22, ainsi que du corps ouvert 21, comporte un chanfrein. Ces chanfreins de la bague et du corps sont désignés en 226 et 216, respectivement. Le chanfrein 226 qui forme une surface conique en saillie axialement de la bague 22, et le chanfrein 216 usiné dans le corps 21 permettent de rendre plus aisé l'engagement dans ce dernier de la bague 22.

Sur la figure 2, la référence 217 désigne un épaulement en saillie du logement formé par les branches 211 et 212, et prévu à l'extrémité du corps en "U" opposée au chanfrein 216, suivant X-X'. Cet épaulement 217 constitue une butée axiale apte à limiter le coulissement de la bague 22, lorsque celle-ci se trouve antérieurement logée entre les branches 211 et 212 du "U" du corps 21.

Suivant le mode de réalisation de la figure 2, le corps 21 est venu de matière avec un crochet d'ancrage 24. Ce crochet est courbé suivant un plan parallèle à l'axe X-X' et fait saillie du corps 21 à l'opposé de l'espace vide entre les surfaces 213 et 214. Le crochet 24 est prévu pour prendre appui contre l'une des vertèbres du rachis R, afin de participer à l'ancrage de l'instrumentation S sur celui-ci.

D'autres modes de réalisation sont illustrés sur la figure 1. Sur cette figure, les ensembles 2 situés à proximité des extrémités supérieures 11 des tiges 1, comportent chacun un doigt recourbé 25. A i'instar du crochet 24, chaque doigt 25 est venu de matière avec le corps 21 correspondant. En fait, le doigt 25 s'étend latéralement depuis l'une des branches du "U" du corps, et est prévu pour venir s'ancrer sur une vertèbre du rachis R. Autrement dit, les doigts 25 permettent un ancrage et un positionnement des tiges 1, perpendiculairement à leurs axes longitudinaux.

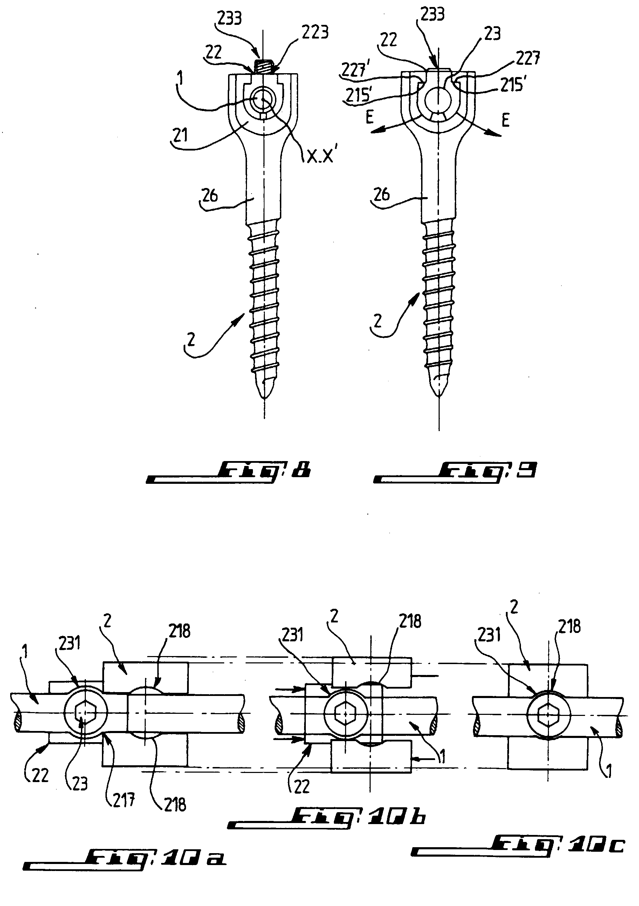

A proximité des extrémités des tiges 1 opposées aux extrémités 11, les ensembles 2 comportent, comme sur les figures 4 et 5, des vis d'ancrage 26, venues de matière avec le corps 21 correspondant. Chacune de ces vis 26 qui s'étend perpendiculairement à X-X' et au droit du fond du "U" du corps 21, est prévue pour venir se loger dans un perçage effectué dans une vertèbre correspondante du rachis à étayer R, afin de constituer un point d'ancrage fixe et solide pour l'instrumentation S.

Les ensembles 2 situés au-dessus de ceux qui comportent les vis 26 (figure 1), permettent de fixer entre les tiges 1 du matériel S une traverse 27 qui joue le rôle d'entretoise.

Ici, la traverse 27 passe à l'intérieur d'orifices (non représentés) ménagés en vis-à-vis dans les branches du "U" de chacun des corps des ensembles 2 correspondants.

Puisque les branches 211 et 212 sont, comme les ailes 221 et 222, élastiquement déformables (similairement à l'écartement indiqué en E sur la figure 5) de manière à s'écarter l'une de l'autre lorsque les vis de pression 23 correspondantes sont serrées, les orifices précités de chaque corps "U" se décalent alors l'un par rapport à l'autre. Ceci provoque l'immobilisation et le blocage de la traverse 27 par arc-boutement de celle-ci dans les orifices dès corps 21. Evidemment, dès que les vis 23 sont desserrées, les orifices correspondants se réalignent et permettent à nouveau un libre coulissement de la traverse 27 à l'intérieur des orifices des corps en "U" reliés par celle-ci.

Avant d'expliquer la manière dont fonctionne le dispositif de fixation conforme à l'invention, un autre ensemble 2 représenté sur la figure 3 va maintenant être décrit.

Comme illustré, cet ensemble comprend un organe constitué par une vis d'ancrage 28 dont la tête forme un manchon fendu 281. Autrement dit, la tête de la vis d'ancrage 28 est en forme de chemise cylindrique filetée intérieurement et divisée en deux moitiés égales par une fente 283, parallèle à l'axe longitudinal de la vis. D'autre part, un perçage (en pointillés) est ménagé dans le "U" du corps 21 correspondant. La vis 28 ainsi qu'une partie du manchon 281 sont insérés dans le perçage du corps 21. On peut également prévoir que la partie de la vis 28 engagée de le perçage du corps 21, ainsi que le perçage lui-même soient légèrement coniques pour obtenir un premier positionnement et un premier blocage axial de la vis 28 par rapport au corps 21. Une fois que la vis 28 est en position, un élément fileté 282 approprié, est vissé dans le filetage du manchon 281. Sous l'effet du serrage de l'élément fileté 282, les moitiés de manchon 281 s'écartent et viennent se plaquer radialement contre le perçage du corps 21, de manière à fixer la vis 28 sur celui-ci.

Sur la figure 3, le perçage du corps 21 dans lequel la vis 28 est montée est réalisée dans l'une des branches du "U" du corps 21, et l'axe de cette vis 28 est sécant à un plan parallèle à X-X' qui divise symétriquement le logement défini par les branches du "U" du corps. Cependant, il va de soi que d'autres dispositions peuvent être prévues pour la disposition de la vis 28, ainsi que du perçage correspondant.

Le montage, le positionnement et l'immobilisation d'un ensemble 2 équipé du dispositif de fixation conforme à l'invention, s'effectuent comme suit.

Tout d'abord, chaque bague 22 est montée sur la tige 1 correspondante' en la déplaçant suivant le mouvement de la flèche M1 sur la figure 2, jusqu'à son clippage sur cette tige. Alors, le corps en "U" 21 approprié est déplacé suivant le mouvement de la flèche M2 sur la figure 2, de façon que la bague 22 vienne se loger dans l'évidement défini par les branches 211 et 212. Dans le cas où le corps 21 comporte un épaulement formant butée 217, le déplacement suivant X-X' du corps 21 est effectué jusqu'à ce que l'extrémité correspondante de la bague 22 vienne en contact avec l'épaulement formant butée 217. Evidemment,la tige 1 et la bague 22 peuvent aussi être déplaçées pour leur assemblage avec le corps 21.

Alors, l'ensemble 2 se retrouve dans une configuration telle que celle illustrée sur la figure 4. C'est seulement à ce moment là que les moyens de serrage constitués par la vis 23 sont actionnés. Ici, la vis 23 est une vis sans tête à six pans creux. Sous l'effet de son serrage, la vis 23 fait saillie entre les ailes 221 et 222, en exerçant une poussée radiale sur la tige 1. Comme illustré sur la figure 5, la poussée exercée par la vis 23 déplace radialement la tige 1 en l'éloignant de la protubérance prismatique 223, de sorte que celle-ci fait saillie de la bague 22, et vient s'appliquer contre le fond du "U" du corps 21. Dans le cas où le corps 21 possède une gouttière 215, c'est contre cette gouttière que la tige 1 vient se plaquer. Ce déplacement de la tige 1 provoque un déplacement simultané et opposé de la bague 22 à l'intérieur du corps 21. Autrement dit, sous l'effet du serrage des moyens 23, la bague 22 est éloignée radialement du fond du corps 21. Lors de ce déplacement, les ailes 221 et 222 glissent sur la périphérie de la tige et s'écartent, jusqu'à ce que le rebord de chacune de leurs extrémités libres viennent pincer une génératrice de la tige 1.

On remarque ici que le contact entre le corps 21 et la tige 1 est au moins linéaire, et surfacique dans le cas où ce corps possède une gouttière 215, tandis que le contact entre la douille fendue 22 et cette tige 1 s'effectue suivant deux génératrices de celle-ci diagonalement opposées. Aussi, en comparaison avec l'art antérieur, les zones de contact avec la tige 1 par l'intermédiaire desquelles son immobilisation s'effectue sont considérablement plus importantes.

Pour que les rebords des extrémités libres des ailes 221 et 222 exercent sur la tige 1 un pincement orienté vers l'axe X-X' sous l'effet du serrage de la vis 23, la partie supérieure externe de chacune de ses ailes, à savoir les surfaces externes les plus proches de la protubérance prismatique 223, coopère avec et est sollicitée par l'une des surfaces correspondantes des branches 211 et 212. La sollicitation de ces surfaces provoquent également la fixation par effet de coin de la bague 22 dans le corps 21.

A cet effet, ces surfaces sont situées au droit des ailes 221 et 222, et convergent l'une vers l'autre. Sur la figure 1 ces surfaces du corps 21 et de la bague 22 ont un profil en arc de cercle, mais on peut également prévoir qu'elles soient planes et convergent vers l'extérieur, ainsi que vers le plan de symétrie de la bague 22.

Il faut noter ici que l'effet de coin obtenu conformément à l'invention et grâce auquel le corps 21 et la bague 22 sont assemblés met en oeuvre des forces et pressions orientées suivant la direction radiale de la tige 1. Ceci est complètement différent de certains dispositifs de l'art antérieur dans lesquels un corps et une douille étaient assemblés en provoquant un effet de coin à l'aide de surfaces tronconiques dont les génératrices convergent vers l'axe de la tige, et donc s'engageant l'une dans l'autre suivant la direction axiale de celle-ci.

On soulignera aussi que les branches 211 et 212 s'écartent légèrement sous l'effet du serrage de la vis 23, comme indiqué en E sur la figure 5.

En outre, dans tous les dispositifs connus, une fois que la douille intermédiaire était montée sur la tige, celle-ci était constamment maintenue et immobilisée sur cette tige. Par contre, avec la présente invention, après le clippage de la bague 22 sur la tige 1, les moyens de serrage provoquent un déplacement radial de cette bague. C'est ce déplacement qui permet d'obtenir l'assemblage du corps 21 et de la bague 22.

Un autre avantage considérable de la présente invention consiste en ce que, même si les moyens de serrage 23 sont desserrés, l'ensemble 2 reste immobilisé sur la tige 1. Par ailleurs, le dispositif conforme à l'invention, se différencie également de l'art antérieur en ce que les efforts appliqués par la vis de serrage 23, les ailes 221 et 222, ainsi que le fond du corps 21 génèrent dans cette tige 1 des efforts convergents vers l'axe X-X' de la tige, et orientés en croix, lorsque le dispositif est observé transversalement, comme sur la figure 5.

La figure 6 montre un deuxième mode de réalisation de l'invention qui se distingue du premier représenté à la figure 2, par le fait que la douille 22 est réalisée sous forme d'une bague fendue sensiblement cylindrique, qui comprend, diamétralement opposée au perçage 223 de la protubérance prismatique 23, une fente axiale d'une largeur relativement faible. Ainsi les ailes latérales 221, 222 de la bague 22 définissent une surface interne sensiblement cylindrique et dont la section correspond à peu près à celle de la tige 1. La bague 22 peut être enfilée axialement sur la tige lisse 1, comme l'indique la flèche M3 sur la figure 6. Dans cette position, les ailes latérales 221 et 222 enserrent la tige 1 à l'intérieur de la bague fendue 22, tout en permettant leur coulissement axial relatif.

Dans ce mode de réalisation la tige sous l'effet des moyens de serrage écarte les extrémités libres les ailes 221, 222 de la bague 22 pour assurer leur plaquage radial contre les branches 211, 212 du corps 21 qui retiennent ainsi la bague dans ce corps par effet de coin, mais reste en appui sur ces extrémités de façon à être immobilisée dans la bague par pincement suivant deux génératrices. Les figures 8 et 9 illustrent cet effet d'immobilisation de la tige dans la bague dans le corps en forme de "U".

La figure 7 montre un mode de réalisation avantageux de l'invention. Dans ce cas aussi, la douille 22 est réalisée sous forme d'une bague fendue qui présente une fente d'une largeur relativement faible, conformément au mode de réalisation selon la figure 6, de façon que les branches 221, 222 délimitent un logement sensiblement cylindrique pour la tige 1. Comme dans le cas de la figure 6, la bague est montée sur la tige par enfilement axial symbolisé par la flèche M3. On constate encore que les branches latérales 211, 212 du corps en forme de "U" 21 définissent un logement sensiblement cylindrique dont la forme correspond à celle de la bague 22.

Une particularité essentielle de ce mode de réalisation réside dans le fait que chaque face plane 224, 224' de la protubérance prismatique 223 de la bague 22 présente dans sa partie axialement centrale une excroissance 231 en forme d'un segment cylindrique. De façon complémentaire, chacune des faces planes 213, 214 des extrémités des branches latérales 211, 212 du corps 21 présente un évidement 218 en forme d'un arc de cercle dans laquelle s'engagera l'excroissance 231 de la bague 22, lorsque ce dernier se trouve dans sa position d'assemblage du dispositif de fixation selon l'invention. De cette manière, la bague est axialement verrouillée dans les deux sens.

On constate encore que la bague 22 comporte à la naissance de chaque face latérale plane 224, 224' de la protubérance prismatique 223 une face d'épaulement plane 227, 227'. Chaque face s'étend sensiblement perpendiculairement à l'axe des moyens de serrage ou est légèrement inclinée par rapport à cet axe en direction de l'axe de la bague. De façon complémentaire, chaque extrémités d'une aile 211 ou 212 du corps 21 comportent dans sa partie interne une face plane 215, 215' formant face d'appui d'une face 227, 227' de la bague, comme le montre les figures 8 et 9. On obtient ainsi un coincement parfait de la douille 22 dans le corps 21 grâce à des formes et pression orientées radialement par rapport à la tige. Les figures 8 et 9 montrent également que grâce à la coopération des surfaces 227 et 227' de la bague et des surfaces 217 et 217' du corps, on évite un écartement les extrémites libres des branches du corps et tout dégagement accidental de la bague, du corps 21.

On notera encore que l'une au moins des extrémités opposées suivant l'axe longitudinal de la bague fendue 22, ainsi que du corps 21, comporte un chanfrein. Ces chanfreins de la bague et du corps sont désignés en 226 et 216 respectivement. Le chanfrein 226 qui forme une surface conique en saillie axialement de la bague 22, et le chanfrein 216 usiné dans le corps 21 permettent de rendre plus aisé l'engagement dans ce dernier de la bague 22.

Les figures 10a à 10c illustrent le montage, le positionnement et l'immobilisation de l'ensemble équipé du dispositif de fixation selon le mode de réalisation de la figure 7. Tout d'abord, chaque bague 22 est montée sur la tige 1 correspondante en la déplaçant suivant la flèche M3 de la figure 7. Puis le corps 21 approprié est déplacé suivant la flèche M4 de la figure 7, de façon que la bague 22 vienne se loger dans l'évidement défini par les branches 211 et 212 jusqu'à ce que les excroissances 231 de la bague 22 soient en contact avec les bords des faces 213, 214 du corps 21. La bague 22 est ensuite introduite par coulissement à force provoquant l'écartement des faces 213 et 214 de façon élastique jusqu'à ce que les surfaces cylindriques convexe des excroissances 231 se placent dans leurs évidements cylindriques concaves complémentaires 218 du corps 21. Les faces 213 et 214 du corps retrouvent alors leur position initiale.

## Revendications

1. Dispositif de fixation sur une tige (1) d'une organe (24, 25, 26, 27, 28) tel que vis, ou crochet ou traverse, notamment pour une instrumentation d'orthopédie rachidienne (S), et comprenant :
- un corps (21) en forme de "U", solidaire dudit organe et entre les branches (211, 212) duquel la tige (1) peut être montée,
- une douille intermédiaire (22) pouvant être montée sur la tige (1) ainsi qu'à coulissement entre les branches du corps (21), et
- des moyens de serrage (23), solidaires de la douille (22) et aptes à exercer une pression radiale sur la tige (1),
caractérisé en ce que la douille présente sensiblement la forme d'une bague fendue (22) comportant des ailes (221, 222) dont les extrémités libres sont écartées l'une de l'autre et qui peut être engagée sur la tige (1) de façon que lesdits moyens de serrage (23) disposés sur la bague (22) soient disposés au droit de la fente de celle-ci, et en ce que la tige sous l'effet de serrage exercé par les moyens de serrage (23) écarte les extrémités libres des ailes (221, 222) de la bague fendue (22) pour que les extrémités libres des ailes (221, 222) soient radialement plaquées, d'une part, contre les branches (211, 212) du "U" du corps (21) qui retiennent ainsi la bague dans ce corps par effet de coin, et d'autre part, contre la tige (1) afin de l'immobiliser par pincement.

2. Dispositif selon la revendication 1, caractérisé en ce que la douille présente la forme d'une bague fendue (22) qui peut être engagée radialement par enserrage élastique ou "clippage" sur la tige (1) et en ce que sous l'effet du serrage exercé par les moyens de serrage (23), la tige (1) fasse saillie de la bague (22) en s'appliquant contre le fond du "U" du corps (21) en produisant les effets de plaquage et d'immobilisation précités.

3. Dispositif selon la revendication 1, caractérisé en ce que le fond du "U" du corps (21) comporte un évidement axial (215) en forme de gouttière et correspondant au diamètre de la tige (1).

4. Dispositif selon la revendication 2, caractérisé en ce que la gouttière (215) précitée présente un état de surface rugueux et/ou des stries transversales, afin d'accroître l'adhérence par frottement entre la tige (1) et le corps (21).

5. Dispositif selon l'une des revendications 1 à 3, caractérisé en ce que l'organe précité est une traverse (27) passant à l'intérieur d'orifices ménagés en vis-à-vis dans les branches (211, 212) du "U" du corps (21), ces branches étant élastiquement déformables (E) sous l'effet de moyens de serrage (23), afin d'immobiliser la traverse (27) sur le corps par arc-boutement.

6. Dispositif selon l'une des revendications 1 à 4, caractérisé en ce que l'organe précité est une vis (28) d'ancrage dont la tête forme un manchon fendu (281) et qui est insérée à l'intérieur d'un perçage ménagé dans le corps (21), la vis étant fixée sur celui-ci à l'aide d'un élément fileté (282) apte à plaquer ledit manchon (281) radialement contre le perçage du corps.

7. Dispositif selon la revendication 5, caractérisé en ce que le perçage précité où la vis d'ancrage (28) est montée, est ménagé dans une branche du "U" du corps (21).

8. Dispositif selon l'une des revendications 1 à 6, caractérisé en ce que l'une au moins des extrémités opposées de la bague fendue (22) ainsi que du corps en "U" (21) précités sont chanfreinés (216, 226).

9. Dispositif selon la revendication 1, caractérisé en ce que la douille (22) présente la forme d'une bague cylindrique fendue engageable sur la tige (1) par enfilement axial, la fente présentant une largeur faible de façon qu'à l'état écarté sous l'effet des moyens de serrage (23,) la tige reste en appui sur les faces internes des extrémités libres des ailes (221, 222), qui délimite la fente.

10. Dispositif selon l'une des revendications précédentes, caractérisé en ce que la douille en forme de bague fendue (22) comprend, diamétralement opposée à la fente, une protubérance radiale extérieure, avantageusement prismatique (223), qui comprend au moins une surface (224 ou 224') parallèle à l'axe longitudinal de la bague et le corps en forme de "U" (21) comprend à l'extrémité d'une de ses branches (211, 212) une face (213 ou 214) plane, paralléle à la face (224, 224') de la bague (22) destinée à venir en contact avec celle-ci, lorsque la bague est montée dans ledit corps, et en ce qu'une des deux faces planes (213, 214 ou 224, 224') comporte une excrosissance (231) et l'autre face comporte un évidement (218) de forme complémentaire, de façon que la bague soit verrouillée axialement dans ce corps par engagement de l'excroissance dans l'évidement.

11. Dispositif selon la revendication 10, caractérisé en ce que la protubérance prismatique (223) comprend deux faces planes (224 et 224') l'une parallèle à l'autre, chacune coopérant avec une surface plane (213 et 214) prévue à l'extrémité d'une branche (211, 212) de corps en forme de "U" (21), chaque paire de surfaces en regard formée par une face plane (224 ou 224') d'une protubérance (223) et une face d'extrémité de branche (211 ou 212) comportant une excroissance (231) et un évidement (218) complémentaires précités.

12. Dispositif selon l'une des revendications 10 ou 11, caractérisé en ce que l'excroissance (231) précitée présente la forme d'un segment cylindrique.

13. Dispositif selon l'une des revendications 11 ou 12, caractérisé en ce que la protubérance prismatique (223) comprend à la base de chaque face plane (224, 224') une face (227, 227') qui est perpendiculaire ou légèrement inclinée vers l'intérieur, par rapport au plan diamétrai médian passant à travers la fente de la bague, et en ce que le corps (21) comporte à l'extrémité de chaque branche( 211, 212) une face (215, 215') adaptée pour constituer une face d'appui des faces (227, 227') de la bague.

## Claims

1. Device for fixing an element (24, 25, 26, 27, 28), such as a screw or hook or crosspiece, on a rod (1), particularly for a spinal orthopaedics instrument (S), and comprising:
- a "U"-shaped body (21) which is integral with the said element and between the branches (211, 212) of which the rod (1) can be mounted,
- an intermediate bushing (22) which can be mounted on the rod (1) and is able to slide between the branches of the body (21), and
- clamping means (23) which are integral with the bushing (22) and are able to exert a radial pressure on the rod (1),
characterized in that the bushing has substantially the shape of a slotted ring (22) including wings (221, 222) whose free ends are spaced apart from one another, and which can be engaged on the rod (1) in such a way that the said clamping means (23) arranged on the ring (22) are at right angles to the slot of the latter, and in that the rod, under the clamping effect exerted by the clamping means (23), spreads the free ends of the wings (221, 222) of the slotted ring (22) apart so that the free ends of the wings (221, 222) are pressed radially, on the one hand, against the branches (211, 212) of the "U" of the body, which thus maintain the ring in this body by a wedging effect, and, on the other hand, against the rod (1) in order to immobilize it by pinching.

2. Device according to Claim 1, characterized in that the bushing has the shape of a slotted ring (22) which can be engaged radially by elastic clasping or clipping on the rod (1), and in that, under the clamping effect exerted by the clamping means (23), the rod (1) protrudes from the ring (22) and bears against the bottom of the "U" of the body (21), thus producing the aforementioned pressing and immobilizing effects.

3. Device according to Claim 1, characterized in that the bottom of the "U" of the body (21) includes an axial recess (215) having a trough shape and corresponding to the diameter of the rod (1).

4. Device according to Claim 2, characterized in that the aforementioned trough (215) has a roughened surface and/or transverse scores in order to increase the frictional adherence between the rod (1) and the body (21).

5. Device according to one of Claims 1 to 3, characterized in that the aforementioned element is a crosspiece (27) passing through orifices formed opposite each other in the branches (211, 212) of the "U" of the body (21), these branches being elastically deformable (E) under the effect of clamping means (23) in order to immobilize the crosspiece (27) on the body by buttressing.

6. Device according to one of Claims 1 to 4, characterized in that the aforementioned element is an anchoring screw (28) whose head forms a slotted sleeve (281) and which is inserted inside a bore formed in the body (21), the screw being fixed on the latter with the aid of a threaded element (282) able to press the said sleeve (281) radially against the bore of the body.

7. Device according to Claim 5, characterized in that the aforementioned bore in which the anchoring screw (28) is mounted is formed in a branch of the "U" of the body (21).

8. Device according to one of Claims 1 to 6, characterized in that at least one of the aforementioned opposite ends of the slotted ring (22) and of the "U"-shaped body (21) are bevelled (216, 226).

9. Device according to Claim 1, characterized in that the bushing (22) has the shape of a slotted cylindrical ring which can be fitted on the rod (1) by axial engagement, the slot having a small width in such a way that in the spaced-apart state, under the effect of the clamping means (23), the rod remains bearing on the internal faces of the free ends of the wings (221, 222), which delimits the slot.

10. Device according to one of the preceding claims, characterized in that the bushing in the shape of a slotted ring (22) comprises, diametrically opposite the slot, an external radial protuberance, advantageously prismatic (223), which comprises at least one surface (224 or 224') parallel to the longitudinal axis of the ring, and the "U"-shaped body (21) comprises, at the end of one of its branches (211, 212), a plane face (213 or 214) parallel to the face (224, 224') of the ring (22) intended to come into contact with it, when the ring is mounted in the said body, and in that one of the two plane faces (213, 214 or 224, 224') includes an excrescence (231) and the other face includes a recess (218) of complementary shape so that the ring is locked axially in this body by means of the excrescence engaging in the recess.

11. Device according to Claim 10, characterized in that the prismatic protuberance (223) comprises two plane faces (224 and 224'), one parallel to the other, each cooperating with a plane surface (213 and 214) provided at the end of a branch (211, 212) of the "U"-shaped body (21), each facing pair of surfaces formed by a plane face (224 or 224') of a protuberance (223) and an end face of a branch (211 or 212) including an aforementioned complementary excrescence (231) and recess (218).

12. Device according to either of Claims 10 and 11, characterized in that the aforementioned excrescence (231) has the shape of a cylindrical segment.

13. Device according to either of Claims 11 and 12, characterized in that the prismatic protuberance (223) comprises, at the base of each plane face (224, 224'), a face (227, 227') which is perpendicular or slightly inclined inwards, in relation to the median diametral plane passing through the slot of the ring, and in that the body (21) includes, at the end of each branch (211, 212), a face (215, 215') adapted to constitute a bearing face of the faces (227, 227') of the ring.

## Patentansprüche

1. Vorrichtung zur Befestigung eines Glieds (24, 25, 26, 27, 28), wie zum Beispiel einer Schraube, eines Hakens oder eines Querglieds, an einer Stange, insbesondere für ein Spinalorthopädie-Instrument (S), wobei die Vorrichtung folgendes umfaßt:
- einen "U"-förmigen Körper (21), der fest mit dem Glied verbunden ist und zwischen dessen Schenkeln (211, 212) die Stange (1) angebracht werden kann,
- eine Zwischenhülse (22), die an der Stange (1) angebracht und zwischen den Schenkeln des Körpers (21) gleiten kann, und
- fest mit der Hülse (22) verbundene Klemmittel (23), die auf die Stange (1) einen Radialdruck ausüben können,
dadurch gekennzeichnet, daß die Hülse im wesentlichen die Form eines gespaltenen Rings (22) mit Flügeln (221, 222) aufweist, deren freie Enden voneinander beabstandet sind, wobei der Ring so an der Stange (1) in Eingriff gebracht werden kann, daß die an dem Ring (22) angeordneten Klemmittel (23) senkrecht zu seinem Schlitz angeordnet sind, und daß die Stange unter der durch die Klemmittel (23) ausgeübten Klemmwirkung die freien Enden der Flügel (221, 222) von dem gespaltenen Ring (22) auseinanderspreizt, damit die freien Enden der Flügel (221, 222) einerseits radial an die Schenkel (211, 212) des "U" des Körpers (21) gepreßt werden, die somit den Ring in diesem Körper durch Keilwirkung festhalten, und andererseits radial an die Stange (1) gepreßt werden, um sie durch Einquetschen festzulegen.

2. Vorrichtung nach Anspruch 1, dadurch gekennzeichnet, daß die Hülse die Form eines gespaltenen Rings (22) aufweist, der durch elastisches Umspannen der Stange (1) oder "Einschnappen" daran mit der Stange in Eingriff gebracht werden kann, und daß die Stange (1) unter der durch die Klemmittel (23) ausgeübten Klemmwirkung aus dem Ring (22) herausragt und so in Anlage an den Boden des "U" des Körpers (21) kommt, wodurch die obengenannte Anpreß- und Festlegungswirkung erzeugt wird.

3. Vorrichtung nach Anspruch 1, dadurch gekennzeichnet, daß der Boden des "U" des Körpers (21) eine rinnenförmige, axiale Aussparung (215) aufweist, die dem Durchmesser der Stange (1) entspricht.

4. Vorrichtung nach Anspruch 2, dadurch gekennzeichnet, daß die obengenannte Rinne (215) eine rauhe oberflächenbeschaffenheit und/oder Querrillen aufweist, um die Haftreibung zwischen der Stange (1) und dem Körper (21) zu erhöhen.

5. Vorrichtung nach einem der Ansprüche 1 bis 3, dadurch gekennzeichnet, daß das obengenannte Glied ein Querglied (27) ist, das sich durch die in den Schenkeln (211, 212) des "U" des Körpers (21) einander gegenüberliegend ausgebildeten Öffnungen erstreckt, wobei diese Schenkel unter der Wirkung von Klemmitteln (23) elastisch verformbar (E) sind, um das Querglied (27) durch Verstrebung an dem Körper festzulegen.

6. Vorrichtung nach einem der Ansprüche 1 bis 4, dadurch gekennzeichnet, daß das obengenannte Glied eine Ankerschraube (28) ist, deren Kopf eine gespaltene Buchse (281) ist und die ins Innere einer im Körper (21) ausgebildeten Bohrung eingesetzt ist, wobei die Schraube mittels eines Gewindeelements (282), das die Buchse (281) radial an die Bohrung des Körpers pressen kann, daran befestigt ist.

7. Vorrichtung nach Anspruch 5, dadurch gekennzeichnet, daß die obengenannte Bohrung, in der die Ankerschraube (28) angebracht ist, in einem Schenkel des "U" des Körpers (21) ausgebildet ist.

8. Vorrichtung nach einem der Ansprüche 1 bis 6, dadurch gekennzeichnet, daß mindestens eines der einander gegenüberliegenden Enden des gespaltenen Ringes (22) sowie des obengenannten "U"-förmigen Körpers (21) abgeschrägt (216, 226) ist.

9. Vorrichtung nach Anspruch 1, dadurch gekennzeichnet, daß die Hülse (22) die Form eines zylindrischen, gespaltenen Rings aufweist, der durch axiales Aufschieben auf die Stange (1) mit dieser in Eingriff gebracht werden kann, wobei der Schlitz eine geringe Breite aufweist, so daß im gespreizten Zustand unter der Wirkung der Klemmittel (23) die Stange an den Innenflächen der freien Enden der Flügel (221, 222), die den Schlitz begrenzen, anliegt.

10. Vorrichtung nach einem der vorhergehenden Ansprüche, dadurch gekennzeichnet, daß die Hülse in Form eines gespaltenen Rings (22) einen vorteilhafterweise prismatischen, äußeren, radialen Vorsprung (223) aufweist, der dem Schlitz diametral gegenüberliegt und mindestens eine Fläche (224 oder 224') aufweist, die parallel zur Längsachse des Rings verläuft, und der "U"-förmige Körper (21) an dem Ende eines seiner Schenkel (211, 212) eine ebene Fläche (213 oder 214) aufweist, die parallel zur Fläche (224, 224') des Rings (22) verläuft, der mit ihr in Berührung kommen soll, wenn der Ring in dem Körper angebracht ist, und daß eine der beiden ebenen Flächen (213, 214 oder 224, 224') eine Ausstülpung (231) und die andere Fläche eine Aussparung (218) komplementärer Form aufweist, so daß der Ring durch Eingreifen der Ausstülpung in die Aussparung axial in diesem Körper verriegelt wird.

11. Vorrichtung nach Anspruch 10, dadurch gekennzeichnet, daß der prismatische Vorsprung (223) zwei ebene Flächen (224 und 224') aufweist, die parallel zueinander verlaufen und jeweils mit einer ebenen Fläche (213 und 214) zusammenwirken, die am Ende eines Schenkels (211, 212) des "U"-förmigen Körpers (21) vorgesehen ist, wobei jedes Paar gegenüberliegender Flächen, das durch eine ebene Fläche (224 oder 224') eines Vorsprungs (223) und eine Endfläche eines Schenkels (211 oder 212) gebildet wird, eine obengenannte Ausstülpung (231) und Aussparung (218), die komplementär sind, aufweist.

12. Vorrichtung nach Anspruch 10 oder 11, dadurch gekennzeichnet, daß die obengenannte Ausstülpung (231) die Form eines zylindrischen Segments aufweist.

13. Vorrichtung nach Anspruch 11 oder 12, dadurch gekennzeichnet, daß der prismatische Vorsprung (223) an der Basis jeder ebenen Fläche (224, 224') eine Fläche (227, 227') aufweist, die bezüglich der diametralen Mittelebene, die durch den Schlitz des Rings verläuft, senkrecht oder leicht nach innen geneigt verläuft, und daß der Körper (21) am Ende jedes Schenkels (211, 212) eine Fläche (215, 215') aufweist, die zur Bildung einer Anlagefläche für die Flächen (227, 227') des Rings ausgeführt ist.
